# EUROPEAN PATENT APPLICATION

(11) **EP 4 640 846 A1**
(43) Date of publication of application: **29.10.2025**
(21) Application number: 23907054.3
(22) Date of filing: 19.12.2023
(51) Int. Cl.: C12Q 1/686

(54) **DNA AMPLIFICATION METHOD USING PCR**

(30) Priority: 23.12.2022 JP 2022206306
(71) Applicant: Peptidream Inc, Kawasaki-ku Kawasaki-shi, Kanagawa 210-0821 (JP)
(72) Inventor: SAWAI, Naoki, Kawasaki-shi, Kanagawa 210-0821 (JP); OHUCHI, Masaki, Kawasaki-shi, Kanagawa 210-0821 (JP); NAKAJIMA, Rui, Kawasaki-shi, Kanagawa 210-0821 (JP)
(74) Representative: Mathys & Squire
(86) International application number: PCT/JP2023/045552
(87) International publication number: WO 2024/135694

(57) **Abstract**

[Problem] To provide a method and a system for amplifying DNA using PCR that can maintain the DNA concentration of a plurality of DNA samples within a certain range.

[Solution] The problem is solved by a DNA amplification method using PCR, the method comprising: a cycle number acquisition step in which the number of PCR cycles for each of DNA samples housed in individual wells of a first multiwell plate is acquired; a minimum PCR step in which PCR for the minimum number of cycles, which is the smallest number of cycles obtained in the cycle number acquisition step, is performed; a first transfer step in which a sample that has been identified with the minimum number of cycles, which is the smallest number of cycles found in the cycle number acquisition step, and that has been subjected to PCR for the minimum cycles is transferred to a second multiwell plate; and a step for repeating additional PCR/transfer steps, in which, after the first transfer step, PCR is performed up to a maximum number of cycles, which is the greatest number of cycles acquired in the cycle number acquisition step, and the sample that has reached the number of cycles acquired in the cycle number acquisition step is transferred to the second multiwell plate.

## Description

### TECHNICAL FIELD

The present invention relates to a method and system for DNA amplification using PCR.

### BACKGROUND OF THE INVENTION

WO 2012/074130 describes mRNA display and RAPID display methods used in peptide translation synthesis.

Various in vitro display techniques utilizing random libraries with nucleic acid tags, such as ribosome display, mRNA display, and RAPID display, generally involve the repetition of the following steps:
Step 1: Generation of a random peptide library with nucleic acid tags
Step 2: Selection of peptides with desired functionality from the random peptide library
Step 3: Amplification of nucleic acids recovered from the selection step to a predetermined concentration (PCR step)

By repeating these three steps, the proportion of peptides with the desired functionality in the peptide library can be increased and enriched, ultimately leading to the acquisition of the target peptide.

Methods and systems have been proposed for normalizing the PCR amplification step for multiple DNA samples in such technologies (WO 2022/081934).

### PRIOR ART LITERATURESSPATENT LITERATURES

Patent Literature 1 WO 2012/074130
Patent Literature 2 WO 2022/081934

### DISCLOSURE OF THE INVENTION

### PROBLEMS TO BE SOLVED BY THE INVENTION

Various displays utilizing random libraries with nucleic acid tags, particularly in the Peptide Discovery Platform System (PDPS) (WO2012/074130), employ in vitro display methods such as mRNA display and RAPID display, where not only the type of random DNA library but also the variations in codon tables and the type of peptide library supplied to the target molecule are combined, resulting in numerous possible combinations. The inventors have considered conducting large-scale screening using nucleic acid tag-based display technology in PDPS to handle a greater number of samples, during which they identified a critical issue arising from the PCR step.

Typically, after the PCR step, the step returns to Step 1. Given the operational considerations of Step 1, it is desirable that all samples have approximately the same concentration after the PCR step. This can be achieved by appropriately determining the number of PCR cycles for each sample in Step 3. However, the nucleic acid tag concentration of the samples recovered from the selection step in Step 2 often exhibits significant variability. Therefore, it is difficult to perform an appropriate PCR cycle for each individual sample of a large number of samples.

To address this issue, grouping samples that require the same or similar cycle numberes and performing PCR within each group is a possible solution. However, conducting such operations with only a few PCR devices results in inefficiency due to repeated execution. Conversely, preparing a large number of PCR devices would require substantial costs and extensive space.

The present specification aims to provide an efficient method and system for DNA amplification using PCR that ensures the concentration of multiple DNA samples remains within a certain range.

### SOLUTIONS TO THE PROBLEMS

The first aspect of the invention relates to a method for amplifying DNA using PCR. This method includes a cycle number acquisition step, a minimum PCR step, a first transfer step, and a repeated additional PCR/transfer step.

The cycle number acquisition step obtains the PCR cycle number for each DNA sample contained in the wells of a first multiwell plate. One example of this step involves acquiring a PCR cycle number such that the amplified DNA concentration falls within a certain range based on the DNA concentration of each sample in the wells. Each DNA sample in the wells preferably contains multiple types of DNA, and these types ideally share identical base sequences at their 5' and 3' ends.

The minimum PCR step performs PCR for the minimum cycle number obtained in the cycle number acquisition step. Before the minimum PCR step, an oil supply unit preferably provides oil to each well of the first multiwell plate, and a reaction liquid supply unit preferably provides reaction liquid to each well. During the minimum PCR step, it is preferable to have the inner lid in a covered state and the movable lid in a heatable state.

The first transfer step transfers samples that underwent PCR for the minimum cycle number from the first multiwell plate to a second multiwell plate. During this step, it is preferable to set the movable lid to a non-heating state and the inner lid to an open state. The wells of the second multiwell plate preferably correspond to those of the first multiwell plate. It is preferable to transfer the sample from each well in the first multiwell plate to the corresponding well in the second multiwell plate.

The step of repeating the additional PCR and transfer step involves performing PCR up to the maximum cycle number obtained in the cycle number acquisition step after the first transfer step, while repeatedly executing the additional PCR and transfer step in which samples that have reached the acquired cycle number are transferred to the second multiwell.

During the additional PCR and transfer step, it is preferable to set the inner lid to a covered state and the movable lid to a heating-enabled state during PCR, and to set the movable lid to a non-heating state and the inner lid to an open state when transferring samples from the first multiwell to the second multiwell. In the additional PCR and transfer step, it is preferable that the sample to be transferred is moved from the well in the first multiwell to the corresponding well in the second multiwell.

The next aspect of the invention relates to a program for amplifying DNA using a PCR device. This program issues commands to a processor to implement the aforementioned steps and directs the PCR device to execute these steps.
In other words, the program enables the processor to:
Execute the cycle number acquisition step,
Direct the PCR device via the processor to perform the minimum PCR step,
Direct the PCR device via the processor to execute the first transfer step,
Direct the PCR device via the processor to repeat the additional PCR/transfer step.
An example of the PCR device will be described in the following sections. Additionally, the invention relates to a non-transitory information storage medium storing the program described above.

The next aspect of the invention pertains to a DNA amplification system using a PCR device.

The DNA amplification system includes a PCR device. The PCR device comprises a first multiwell plate, a second multiwell plate, a sample transfer unit, a thermal cycler, and a processor. Preferably, the wells of the second multiwell plate correspond to those of the first multiwell plate. The device preferably includes a movable lid and an inner lid. The movable lid can be in a heatable state to cover the first multiwell plate and can transition to a non-heating state when not covering it. The inner lid can be in a covered state, in which it covers the first multiwell, and can be transitioned from the covered state to an open state, in which it does not cover the first multiwell, and when the movable lid is in a heatable state and the inner lid is in the covered state, the inner lid is positioned between the movable lid and the first multiwell. Preferably, the device further includes an oil supply unit and a reaction liquid supply unit.

The oil supply unit supplies oil to each well of the first multiwell plate, while the reaction liquid supply unit supplies reaction liquid to each well of the first multiwell plate.

The PCR device implements the method described above and operates as follows: PCR cycle numbers are input for each DNA sample contained in the wells of the first multiwell plate of the PCR device.

The processor then drives the thermal cycler containing the first multiwell plate and directs the PCR device to perform PCR for the minimum cycle number acquired in the cycle number acquisition step.

The processor uses the sample transfer unit to transfer samples that underwent PCR for the minimum cycle number from the first multiwell plate to the second multiwell plate. The processor drives the thermal cycler equipped with the first multiwell to perform additional PCR up to the maximum cycle number entered in the cycle number input step, while repeatedly executing the additional PCR and transfer step using the sample transport unit to transfer the samples that have reached the cycle number entered in the cycle number input step to the second multiwell.

Since the method of the present invention includes the aforementioned steps, the thermal cycler alternates between open and closed states during operation.

### EFFECTS OF THE INVENTION

The present invention enables the DNA concentrations of multiple distinct samples subjected to PCR to be maintained within a specific range. As a result, subsequent processing steps can proceed efficiently using these samples.

Additionally, because this method eliminates the need for dilution, DNA amplification can be performed while preserving the diversity of DNA in samples, particularly in mRNA display techniques using nucleic acid-tagged random libraries, allowing the amplified DNA to be effectively utilized in the next processing stage.

This method is easily adaptable to automation in in vitro display techniques that utilize nucleic acid-tagged random libraries.

This method does not require multiple PCR devices, yet enables rapid processing of numerous samples while ensuring uniform DNA concentration within a designated range.
In addition, this method effectively reduces the risk of contamination between different samples while optimizing the PCR step according to each sample's concentration.

### BRIEF DESCRIPTION OF THE DRAWINGS

[FIG. 1A] Figure 1A illustrates a DNA amplification system with an active sample transfer unit.
[FIG. 1B] Figure 1B depicts a DNA amplification system with the inner lid in a covered state.
[FIG. 1C] Figure 1C shows a DNA amplification system with the movable lid in a heatable state.
[FIG. 2] Figure 2 provides a block diagram of the processor.
[FIG. 3] Figure 3 presents a flowchart explaining the DNA amplification method using PCR.
[FIG. 4] Figure 4 conceptually illustrates the PCR cycle number for each DNA sample contained in the wells of the first multiwell plate.
[FIG. 5A] Figure 5A displays a conceptual diagram of the inner lid being lifted.
[FIG. 5B] Figure 5B shows a conceptual diagram of the step of collecting DNA samples following PCR cycles.
[FIG. 5C] Figure 5C conceptually illustrates the transfer of collected DNA samples to corresponding wells in the second multiwell plate.
[FIG. 5D] Figure 5D illustrates the movement of the inner lid onto the thermal cycler.
[FIG. 5E] Figure 5E depicts the inner lid positioned on the thermal cycler.
[FIG. 5F] Figure 5F displays the movable lid in a heatable state.
[FIG. 6] Figure 6 provides an alternative photograph illustrating microchannel electrophoresis images in Example 2.

### DETAILED DESCRIPTION OF THE INVENTION

The following describes embodiments for implementing the invention with reference to the drawings. The invention is not limited to these embodiments, and variations that are evident to those skilled in the art based on these examples are also included.

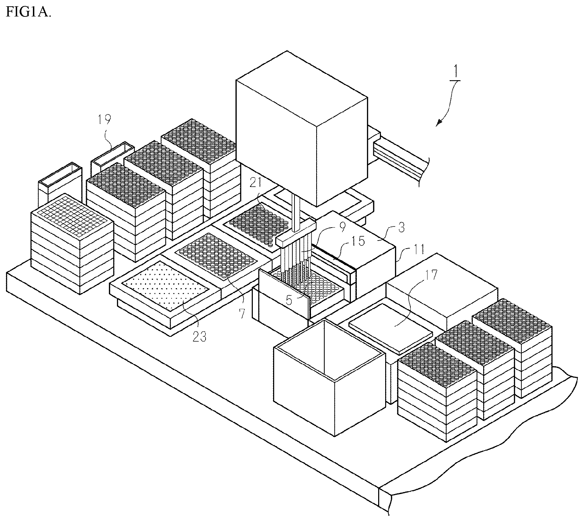

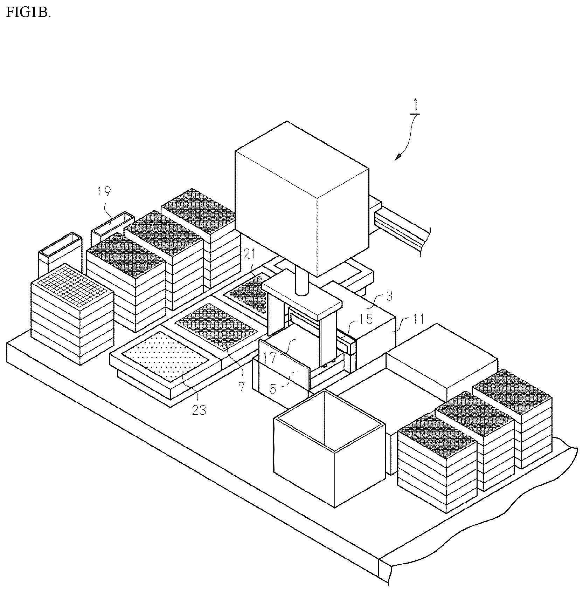

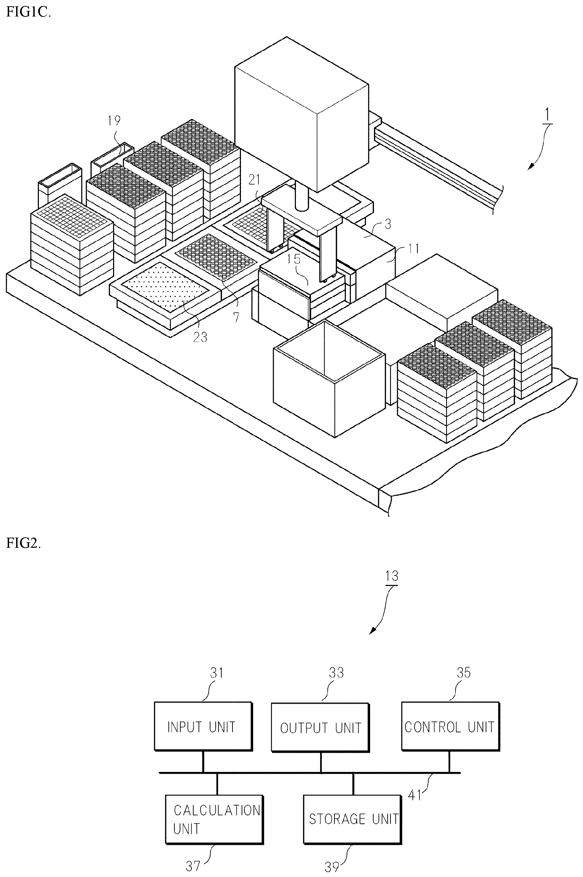

FIG. 1 illustrates a DNA amplification system. FIG. 1A shows the operation of the sample transfer unit. FIG. 1B depicts the system with the inner lid in a covered state. FIG. 1C shows the system with the movable lid in a heatable state. The system 1 relates to a DNA amplification system using a PCR device 3. The DNA amplification system 1 comprises the PCR device 3, which includes a first multiwell plate 5, a second multiwell plate 7, a sample transfer unit 9, a thermal cycler 11, and a processor 13. Preferably, the wells of the second multiwell plate 7 correspond to those of the first multiwell plate 5. Additionally, the system 1 may include a movable lid 15 and an inner lid 17. The system 1 may also include an oil supply unit 19 and a reaction liquid supply unit 21.

The system 1 may be connected to a PCR cycle determination device that determines the PCR cycle number for each DNA sample. The PCR cycle determination device may be connected to a concentration analysis unit that analyzes the sample concentration. The PCR cycle determination device can determine the cycle number for each sample based on its concentration. The system 1 can, for example, receive PCR cycle number data and other PCR-related information from the PCR cycle determination device.

### PCR Device 3

A PCR device is an apparatus used to amplify DNA via polymerase chain reaction. PCR devices, such as those disclosed in Patent Publication No. 7038221, are known in the art. The PCR device 3 in the present invention is designed to be compatible with system 1 and implement the DNA amplification method disclosed herein. The PCR device 3 comprises a first multiwell plate, a second multiwell plate, a sample transfer unit, a thermal cycler, and a processor.

### First Multiwell Plate 5

A multiwell consists of two or more wells. The multiwell may be a plate (multiwell plate) containing two or more wells. Multiwell plates are standardized formats for processing and analyzing multiple samples and can vary in form, size, and shape; however, they are typically manufactured in standard sizes and shapes with a standard well arrangement. Examples of well arrays include a 6-well plate (3×2 array of wells), a 12-well plate (4×3 array of wells), a 24-well plate (6×4 array of wells), a 48-well plate (8×6 array of wells), a 96-well plate (12×8 array of wells), and a 384-well plate (24×16 array of wells). The multiwell plates used in the present invention are not specifically limited as long as they are compatible with PCR. The preferred number of wells ranges from 2 to 1000, ideally between 6 and 500. Although not limited thereto, 48-well plates and 96-well plates can be suitably used.
The first multiwell plate 5 typically contains samples for PCR processing in a thermal cycler. Each well 5a, 5b, and 5c of the first multiwell plate 5 is capable of holding DNA samples. PCR processing can be performed even if not all wells of the first multiwell plate 5 contain DNA samples.

### DNA Samples

The DNA samples used in the present invention contain at least one type of DNA, preferably multiple types. The amount of DNA should be sufficient for PCR amplification, but is otherwise not restricted.
Each well may contain samples dispensed from the same DNA sample, but it is preferable that each well holds a different DNA sample. However, this does not exclude the possibility that some wells may contain samples dispensed from the same DNA sample. Here, the term "different DNA sample" refers to a sample that is not dispensed from the same DNA sample. The lower limit for the number of DNA sample types is preferably 2 or more, and may be 10, 20, 30, or even 40 or more. The upper limit is not specifically restricted but should be within the processing capacity of the PCR device, such as 1000, 500, 200, or 100 samples. The number of different DNA sample types is preferably within the number of wells available in the selected multiwell plate. The volume of the DNA sample used in the present invention need only be an amount sufficient for amplification by PCR and can be appropriately determined according to the well volume. Without limitation, in the case of a 96-well multiwell plate, the volume may range from 20 µL to 100 µL.

When selecting peptide molecules that bind to specific targets, screening from random peptide libraries is a widely used approach. Various in vitro display techniques, such as ribosome display and mRNA display, which utilize a cell-free translation system, are particularly advantageous as they enable the rapid construction and screening of highly diverse libraries within a single tube.

The in vitro display method refers to a system in which the phenotype is displayed on the corresponding genotype by linking them via non-covalent or covalent bonds, thereby enabling the enrichment and amplification (selection) of active species using a replication system reconstructed in a test tube. In vitro display methods include ribosome display, mRNA display (WO98/31700), cDNA display, photo-crosslinked cDNA display (WO2016/159211), TRAP (transcription-translation coupled with association of puromycin linker) display (T. Ishizuka et al., Am. Chem. Soc. 2013, 135, 14, 5433-5440), cDNA TRAP display (T. Kondo et al., Chem. Commun., 2021, 57, 2416-2419), and RAPID display (WO2011/049157). In these in vitro display methods, when selecting molecules of peptides or proteins with functional properties from the library, the corresponding gene is linked, allowing for easy reading of its sequence, which is useful for selecting the genetic information of peptides with specific functions.

The following steps are generally repeated in these display techniques:
Step 1: Generation of a random peptide library with nucleic acid tags
Step 2: Selection of peptides that interact with the target molecule
Step 3: PCR amplification of nucleic acids recovered from the selection step
In Step 1, a random DNA library is first prepared, followed by transcription into a random RNA library, which is then translated into a random peptide library.

In Step 2, desired functional or characteristic peptides are selected from the peptide library using an appropriate screening system. To obtain peptide molecules that bind to a specific protein, for example, the peptide pool can be brought into contact with a solid-phase magnetic carrier containing the target protein, and the bound peptide mixture can be collected using a recovery magnet. Additionally, peptides can be screened by passing the peptide population through a column immobilized with the target protein, followed by the recovery of peptide molecules that bind to the column. At this stage, in vitro display technology ensures that each peptide molecule is tagged with its template nucleic acid molecule. For instance, in an mRNA display library, each peptide molecule is attached to an mRNA. The recovered peptide-mRNA complex is then converted back into DNA using reverse transcriptase and supplied to step 3. Alternatively, reverse transcription can be performed before selection to convert the nucleic acid portion into a double-stranded DNA/RNA hybrid.

In Step 3, recovered DNA is amplified using PCR, resulting in an enriched library containing clones with the targeted functionality.

By iterating these steps, the proportion of peptides with desired functions increases, ultimately allowing retrieval of the target peptide.

Here, the distinct DNA sample may include the nucleic acid recovered from the selection step in step 3. That is, the distinct DNA sample may contain the DNA encoding the peptides selected in step 2, in other words, the peptides identified from a random peptide library as interacting with a target substance.

When mRNA is used as the nucleic acid tag, the DNA obtained by reverse transcription thereof may be utilized as the DNA sample. Specifically, an example of a DNA sample is the DNA encoding peptides recovered from the selection step of the mRNA display method. Another example of a DNA sample is the DNA recovered from peptides possessing a DNA tag. Such DNA samples are those recovered during the selection steps of various in vitro display methods. DNA samples containing DNA tags recovered by the above methods typically exhibit varying concentrations. By employing the methods described in this specification, such various types of DNA samples can be amplified to comparable concentrations.

It is preferable that the DNA sample includes a nucleotide sequence in which the nucleotides at the 5' and 3' ends of the sense strand of the DNA are fixed. In other words, this means that the nucleotide sequence also includes fixed nucleotides at the 5' and 3' ends of the antisense strand. That is, each well (5a, 5b, 5c) of the first multiwell 5 preferably contains multiple types of DNA samples in which the nucleotide sequences at the 5' ends are common, and the nucleotide sequences at the 3' ends are also common. Specifically, DNA samples obtained via in vitro display utilizing a random library with nucleic acid tags preferably have common nucleotides at the 5' and 3' ends for each DNA sample contained within a single well. That is, it is preferable that the nucleotides at the 5' ends of multiple DNA samples are identical, and the nucleotides at the 3' ends of multiple DNA samples are also identical. In such cases, a single well contains multiple types of DNA samples, and the intermediate region of nucleotides, situated between the 5' and 3' end nucleotides, preferably contains randomized nucleotides that vary between different DNA samples. Even in this case, if the wells differ, the nucleotide sequences at the 5' and 3' ends may also differ.

An example of the nucleotide at the 5' end of the sense strand of DNA, though not limited thereto, is a nucleic acid containing a promoter sequence for transcription, such as a T7 promoter sequence. By employing this configuration, transcription of DNA can be performed in the subsequent step following PCR. An example of the nucleotide at the 3' end of the sense strand of DNA is a nucleic acid encoding a linker that connects puromycin with the peptide subject to selection. Alternatively, a substance capable of inhibiting the translation step and facilitating the binding of nucleic acids to peptides, similar to puromycin, may be used as a substitute for puromycin.

Randomization refers to the step of designing a nucleic acid segment with theoretically arbitrary sequences across a predefined length. The length of a randomized sequence segment may range from 3 to 60 nucleotides. A chemical or enzymatic reaction that generates random sequence segments may not necessarily produce mathematically random sequences due to potential unknown biases or nucleotide selection. A random peptide library is constructed using diverse peptides encoded by randomized nucleotide sequences, and by binding these peptides to target proteins, a selection system is established to isolate the desired peptides, which is employed in various in vitro display methods utilizing a random peptide library with nucleic acid tags. Therefore, in embodiments employing the method of the present invention in in vitro display utilizing a random library with nucleic acid tags, it is preferable that the DNA sample contains a diverse array of DNA molecules having the aforementioned structure.

Furthermore, in peptide drug discovery development platform systems such as PDPS (Peptide Discovery Platform System) (WO2012/074130), the Flexizyme technology (WO2007/066627) is utilized in Step 1. This step links codons within the DNA samples to non-natural amino acids based on a codon table, thereby constructing random peptide libraries. Following a selection step utilizing this random peptide library, the recovered DNA sample containing DNA tags may include not only various types of random DNA libraries but also combinations arising from different codon tables and types of target substances used in the peptide library. These DNA samples are well-suited for application in the present invention's method. Preferably, the PCR amplification step in the invention occurs at the same well position in a multiwell plate as the peptide selection step. As described later, the recovered samples are ideally transferred to a new multiwell plate of the same type, maintaining correspondence between well positions. That is, in a series of steps within mRNA display utilizing a random library with DNA tags, particularly in PDPS, consistently conducting assays in the same well position within a multiwell plate is preferable from the perspectives of contamination prevention and assay management, and constitutes a preferred embodiment of the present invention.

The first multiwell plate 5 preferably contains multiple distinct DNA samples. The multiple distinct DNA samples may not necessarily undergo processing to equalize their concentrations.

### Second Multiwell Plate 7

The second multiwell plate 7 is similar to the first multiwell plate 5. Each well 7a, 7b, and 7c in the second multiwell plate 7 corresponds to wells 5a, 5b, and 5c in the first multiwell plate 5. For instance, if wells 5a, 5b, and 5c in the first multiwell plate 5 are located at positions A1, A2, and B2, then the corresponding wells 7a, 7b, and 7c in the second multiwell plate 7 should also be located at positions A1, A2, and B2. The well positions correspond to coordinate points (x, y) in a grid format.

The second multiwell plate 7 is preferably mounted on a recovered sample mounting section that accommodates the second multiwell plate. The recovered sample mounting section preferably includes a cooling element for cooling the second multiwell plate 7, which is a plate. The cooling element is preferably connected to a processor. The cooling element can adjust the temperature based on instructions from the processor, thereby cooling the second multiwell plate 7 mounted on the recovered sample mounting section. Examples of such cooling elements include cooling mechanisms employing circulators or Peltier elements. By cooling the second multiwell plate 7, evaporation of the recovered sample can be suppressed.

### Sample Transfer Unit 9

The sample transfer unit 9 injects samples into wells and retrieves them. It comprises pipettes or micro-pipettes, a transport mechanism, and a suction/discharge mechanism for regulating air pressure within the pipettes. The transport mechanism adjusts the position (horizontal and vertical) of the pipettes based on processor commands, while the suction/discharge mechanism controls the intake and release of samples. The transferred volume of samples is preferably adjustable via processor commands. Ideally, the sample transfer unit 9 features multiple micro-pipettes. Pipettes used for injecting and retrieving samples may be identical or distinct components. Additionally, micro-pipettes with an automated tip replacement mechanism are preferred.

### Thermal Cycler 11

The thermal cycler 11 regulates PCR temperature conditions. During the PCR step, the first multiwell 5 is mounted on the plate-mounting section of the thermal cycler 11. The thermal cycler 11 adjusts the temperature of the temperature control unit based on processor commands and is capable of controlling the temperature of the first multiwell 5.

### Processor 13

The processor 13 is a component responsible for issuing commands to various elements in the PCR device 3. Figure 2 is a block diagram illustrating the processor. The processor 13 includes an input unit 31, an output unit 33, a control unit 35, a computation unit 37, and a storage unit 39, and the elements are connected via a bus 41 or the like so as to enable the exchange of information. For example, the storage unit may store programs and various types of information. The processor 13 retrieves programs and performs various computations and processes. That is, the operations performed by the processor 13 may be based on programs. Additionally, the processor 13 may include various circuits such as arithmetic circuits. When predetermined information is input via the input unit, the control unit retrieves programs stored in the storage unit. Subsequently, the control unit appropriately retrieves stored information from the storage unit and transmits it to the computation unit. Furthermore, the control unit transmits appropriately input information to the computation unit. The computation unit performs calculations using the received information and stores the results in the storage unit. The control unit retrieves computed results stored in the storage unit and outputs them via the output unit. In this manner, various steps and operations are executed. The components and means responsible for executing these various steps are each respective unit or mechanism. A computer may be employed to implement various functions and steps. The computer may operate as a standalone system. The computer may have its functions partially distributed between a server and a terminal. In such cases, it is preferable that the server and terminal are configured to exchange information via networks such as the Internet or an intranet. The processor may include pre-trained models obtained through various machine learning processes. In this case, inputting predetermined training data into the pre-trained model can enhance the accuracy of the model and the machine learning process. By inputting various types of data into the pre-trained model, the desired output can be obtained. Examples of such data include PCR processing information and various data necessary for determining DNA concentration.

The processor 13 receives PCR processing information. The PCR processing information includes data regarding the number of PCR cycles for each DNA sample housed in the individual wells 5a, 5b, and 5c of the first multiwell 5. Preferably, the PCR processing information also includes the positional data of wells 5a, 5b, and 5c. The entered PCR processing information is stored in a memory unit and used during the PCR step. Additionally, various types of information necessary for determining DNA concentration may be input into processor 13.

### Movable Lid 15

The movable lid 15 can be set to a heatable state in which it covers and heats the first multiwell 5, and by moving from the heatable state, it can be set to a non-heatable state in which it does not cover the first multiwell 5. The movable lid 15 is an element of the thermal cycler 11, and preferably includes a heating element to enable the heating of the first multiwell 5. Additionally, the movable lid 15 preferably includes a movable lid body containing a heating element and a driving unit such as an actuator to move the movable lid body. Preferably, the heating element and the driving unit are connected to the processor 13. The heating element can adjust the temperature based on commands from the processor 13. The driving unit can change the position of the movable lid 15 based on commands from the processor 13. In this example, the movable lid 15 including a heating element has been described. However, the mounting section that accommodates the first multiwell 5 may have a heating element as part of the thermal cycler 11. While this example describes movable lid 15 as containing a heating element, an alternative configuration would be a mounting platform within thermal cycler 11 that incorporates its own heating elements.

An example of the heating element is an electrical heating wire (resistance wire) arranged across the area of movable lid 15 that covers the wells.

### Inner Lid 17

The inner lid 17 can be set to a covered state in which it covers the first multiwell 5. Preferably, the inner lid 17 is independent of the thermal cycler 11 and can be freely moved. The inner lid 17 can be moved from the covered state to an open state in which it does not cover the first multiwell 5. When the movable lid 15 is in a heatable state and the inner lid 17 is in a covered state, the inner lid 17 is positioned between the movable lid 15 and the first multiwell 5. Preferably, when the inner lid 17 is in the covered state, it can seal the first multiwell 5. The inner lid 17 can be repositioned using an actuator such as an inner lid transport mechanism (also referred to as a handling unit). The inner lid transport mechanism is preferably connected to the processor. An example of the inner lid transport mechanism is a robotic arm equipped with a gripping unit. The inner lid transport mechanism receives commands from the processor 13, grips the inner lid 17 using the gripping unit, changes the position of the gripped inner lid 17, and releases the gripping unit to place the inner lid 17 at a designated location. Since the inner lid 17 exists and can be set to a covered state in which it covers the first multiwell 5, it can prevent the evaporation of liquid inside each well during the PCR processing steps. Furthermore, by using the inner lid 17, cross-contamination between wells can be prevented.

Inner lid 17 is preferably made from highly thermally conductive materials. Example materials include silicon, rubber, and polystyrene. It is particularly beneficial for inner lid 17 to be an automatic sealing polymer lid controlled by processor 13 to maintain a sealed state during PCR processing. Automatic sealing polymer lids minimize reagent evaporation during long incubations. Alternatively, inner lid 17 may be a seal attached to a multiwell plate. The seal is preferably attachable using a sealer and removable using a peeler.

### Oil Supply Unit 19

The oil supply unit 19 is a component for supplying oil to each well of the first multiwell 5. The oil supply unit 19 includes a pipette or similar tube, a transport mechanism for moving the tube, a aspiration and discharge mechanism for adjusting the air inside the tube to perform suction and discharge, and an oil source. The transport mechanism changes the position of the tube in response to commands from the processor. The aspiration and discharge mechanism receives commands from the processor 13 to suction oil from the oil source and discharge it at a predetermined location. Preferably, the amount of oil suctioned and discharged can be adjusted according to commands from the processor. The suctioned oil may be stored in a container (oil source) capable of holding oil for PCR reactions, and preferably, such a container has an open top allowing extraction using a pipette or similar device. Using oil can prevent the evaporation of liquid containing DNA samples when heated during the PCR step. The oil is preferably a high-temperature stable, non-volatile liquid that does not affect the PCR reaction and has a lower specific gravity than the PCR reaction solution. Such oil may be one that is typically used for PCR.

Examples of oil include mineral oil, vegetable oil, animal oil, fluorinated oil, silicone-based oil, and hydrocarbon-based oil. Among these, mineral oil is preferable. The oil volume added should be sufficient to prevent evaporation, and for a 96-well microplate, amounts ranging from 5 µL to 100 µL are suitable. In this invention, inner lid 17 covers the first multiwell plate 5 during PCR processing while oil is added to each well, effectively preventing evaporation even during repeated additional PCR and transfer steps. The micropipette system in oil supply unit 19 should ideally feature an automatic tip replacement mechanism.

In a preferred embodiment, the present invention comprises the aforementioned movable lid 15 with a heating element and the aforementioned oil supply unit 19. It is widely known in PCR that adding oil to a tube containing a sample prevents the reaction solution from evaporating and concentrating. However, this method has certain drawbacks, such as the inconvenience of handling the oil overlay and the necessity of leaving a portion of the sample to prevent oil carryover. To address these issues, a lid with a heating element (heat lid) was developed. As a result, when using a thermal cycler with a heating lid, the use of oil becomes unnecessary, thereby resolving the aforementioned issues. Typically, when using a thermal cycler with a heating lid, opening and closing the heating lid during the DNA amplification step is not anticipated.

To address these challenges, heated lids were developed, eliminating the need for oil. Standard PCR processes using heated lids do not require lid opening during amplification. However, the method of the present invention is characterized in that the open state and the closed state of the movable lid 15 are repeatedly alternated during the DNA amplification step. The inventors have found that, in the method described in this specification, even when the movable lid of the thermal cycler 11 is repeatedly placed in an open state, the PCR step of the present invention can be carried out by preferably using oil or an inner lid 17, and more preferably by using both oil and the inner lid 17, in combination with the movable lid 15.

### Reaction Liquid Supply Unit 21

The reaction liquid supply unit 21 is an element for supplying reaction liquid to each well of the first multiwell 5. The reaction liquid supply unit 21 includes a tube such as a pipette, a transfer mechanism for transporting the tube, a aspiration and discharge mechanism for adjusting the air within the tube to enable suction and discharge, and a reaction liquid source. The transfer mechanism changes the position of the tube in response to instructions from the processor. The aspiration and discharge mechanism, in response to instructions from the processor, suctions the reaction liquid from the reaction liquid source at a predetermined position and discharges the reaction liquid. It is preferable that the amount of reaction liquid suctioned and the amount of reaction liquid discharged can be adjusted according to instructions from the processor 13. The reaction liquid to be suctioned may be retained in a container (reaction liquid source) capable of holding the reaction liquid, and it is preferable that such a container has an open top so that it can be collected using a pipette or similar instrument. The reaction liquid preferably contains primers for the sequence to be amplified by the PCR step, dNTPs (deoxynucleoside triphosphates) serving as substrates for ATGC, and a PCR buffer. The reaction liquid preferably contains salts such as MgCl₂ and KCl. Alternatively, the components used in the PCR reaction may be pre-included in the DNA sample so that they are not contained in the reaction liquid. The forward primer and reverse primer for PCR preferably anneal to the base sequence at the 5' end and the base sequence at the 3' end, respectively. The length of the primers may be a length typically used in PCR, for example, 20 to 60 bases. A person skilled in the art can appropriately design such primers. By using such designed primers, all diverse DNA contained in multiple different DNA samples can be targeted for PCR amplification. The reaction liquid preferably contains at least a thermostable DNA polymerase. The thermostable DNA polymerase may preferably be a known one used in PCR, such as Taq polymerase.

Before reaction liquid addition, thermal cycler 11 may conduct a pre-incubation step with DNA samples. Reaction liquid volume should be consistent with standard PCR requirements.

The micropipettes in reaction liquid supply unit 21 should preferably feature an automatic tip replacement mechanism.

### Magnetic Bead Recovery Magnet 23

Selected nucleic acids may be purified using a method that employs magnetic beads and a recovery magnet, followed by elution into a suitable solvent before undergoing the amplification step described in this invention. Preferably, this entire step should be performed in the same well to ensure continuity.

The present invention relates to a program for amplifying DNA using a PCR device 3. This program causes the processor 13 to issue instructions for implementing various steps and causes the PCR device 3 to execute the various steps. Specifically, this program causes the processor 13 to execute a cycle number input step. Furthermore, this program causes the PCR device 3, via the processor 13, to execute a step in which a minimum PCR step, a first transfer step, and an additional PCR and transfer step are repeated. In addition, the present invention relates to a non-transitory information recording medium storing the aforementioned program. Examples of the non-transitory information recording medium include CD-ROMs, DVDs, SD cards, and USB memory devices.

### Operation of PCR Device 3

The PCR device 3 operates as follows, for example. PCR cycle numbers are input for each DNA sample contained in the wells of the first multiwell plate 5 of the PCR device 3. The processor 13 then drives the thermal cycler 11, which holds the first multiwell plate 5, and directs the PCR device 3 to perform PCR for the minimum cycle number obtained in the cycle number input step. The processor 13 then uses the sample transfer unit 9 to transfer samples that underwent PCR for the minimum cycle number from the first multiwell plate 5 to the second multiwell plate 7.

The processor 13 continues executing PCR using the thermal cycler 11 until reaching the maximum cycle number acquired in the cycle number input step. It further directs the sample transfer unit 9 to transfer samples to the second multiwell plate 7 upon achieving their designated cycle number, repeating the additional PCR and transfer steps. These operations are explained in greater detail in the method for DNA amplification using PCR described below.

### Method for DNA Amplification Using PCR

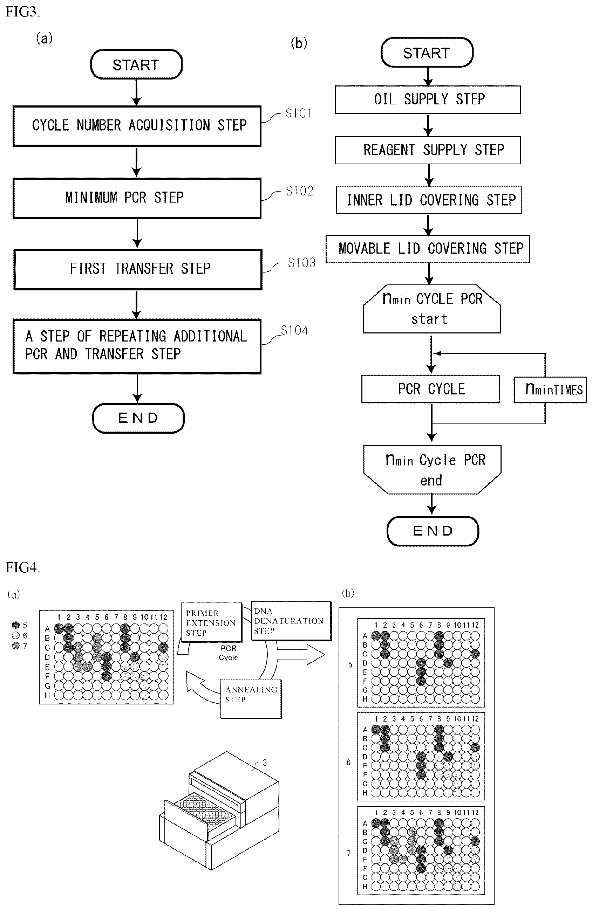

Figure 3 is a flowchart illustrating the method for DNA amplification using PCR. Figure 3(a) shows the overall flow, and Figure 3(b) illustrates the minimum PCR step (S102). As shown in Figure 2, the method for DNA amplification using PCR includes a cycle number acquisition step (S101), a minimum PCR step (S102), a first transfer step (S103), and a step of repeating additional PCR and transfer steps (S104).

### Cycle Number Acquisition Step (S101)

The cycle number acquisition step is a step for acquiring the PCR cycle number for each DNA sample contained in the wells of the first multiwell plate 5. The PCR cycle number for each DNA sample contained in the wells of the first multiwell plate 5 is input into the system 1. In this manner, the system 1 can obtain the PCR cycle number for each DNA sample contained in the wells of the first multiwell plate 5. This step may include a PCR cycle number determination step.

### PCR Cycle Number Determination Step

This step establishes the PCR cycle number needed for each DNA sample in first multiwell plate 5 wells. Specifically, it determines how many PCR cycles are required to achieve a standardized DNA concentration across all wells.

For example, the PCR cycle number determination device determines the PCR cycle number for each DNA sample contained in the wells of the first multiwell plate 5. An example of the cycle number acquisition step is acquiring a PCR cycle number such that the DNA concentration after amplification falls within a predetermined range, based on the DNA concentration of each DNA sample contained in the wells. A known method may be appropriately used to determine the DNA concentration. For example, the DNA concentration can be determined using the quantitative PCR method (qPCR method). The PCR cycle number determination device, in response to an instruction from the processor, operates a liquid transport device such as a micropipette to extract a portion of each DNA sample contained in the wells of the first multiwell plate 5. The PCR cycle number determination device then discharges a portion of the extracted DNA sample into a concentration measurement device based on the quantitative PCR method (qPCR method) and determines the DNA concentration in accordance with the qPCR method, in response to an instruction from the processor. Next, the PCR cycle number determination device, in response to a program instruction, causes the processor to read out information stored in the memory unit necessary for determining the PCR cycle number and perform a calculation to determine the PCR cycle number based on the DNA concentration. An example of the information necessary for determining the PCR cycle number is data that records the relationship between the numerical range of DNA concentration and the PCR cycle number. For example, if the DNA concentration falls within a predetermined range, the PCR cycle number is set to a specified value. The PCR cycle number determined in this manner for each DNA sample contained in the wells of the first multiwell plate 5 is stored in the memory unit as appropriate. The processor may output information regarding the PCR cycle number stored by the PCR cycle number determination device to system 1 or to the PCR device 3 of system 1. Additionally, system 1 and the PCR cycle number determination device may share a processor. This method can be preferably used for mRNA display and RAPID display utilizing random peptide libraries based on DNA tags. By using the quantitative PCR method (qPCR method), the DNA concentration can be determined even when the DNA sample has a low concentration. The PCR device 3 that performs the cycle number acquisition step (S101), the minimum PCR step (S102), the first transfer step (S103), and the step of repeating additional PCR and transfer steps (S104) may be the same as or different from the PCR device used for performing the quantitative PCR method. If these devices are different, the control unit or processor of the PCR device used for the quantitative PCR method may output PCR information and the DNA concentration of each sample to the control unit or processor of the PCR device 3 or system 1.

The PCR cycle number for each well may be determined by setting a target DNA concentration after amplification and calculating the PCR cycle number based on the DNA concentration of each DNA sample contained in the well using PCR amplification efficiency. In this case, the memory unit of the processor stores the PCR amplification efficiency and the target DNA concentration (or the numerical range of the target DNA concentration). The PCR cycle number determination device (or its processor) may read out the DNA concentration of each DNA sample contained in the well, the PCR amplification efficiency, and the DNA concentration (or the numerical range of the target DNA concentration) based on a program instruction, and cause the calculation unit to perform a computation to determine the PCR cycle number. In this manner, the PCR cycle number determination device can determine the PCR cycle number for each well. Preferably, the PCR cycle number is set so that the amount of PCR product does not reach the plateau phase. Therefore, the processor may determine the amount of PCR product based on a program instruction, read out the amount of PCR product corresponding to the plateau phase from the memory unit, and compare these values. If the amount of PCR product exceeds the plateau threshold, the processor may perform a computation to decrement the PCR cycle number by one.

The target DNA concentration is preferably the same value for multiple DNA samples with different concentrations. Furthermore, the target DNA concentration may be appropriately adjusted depending on the intended use of the amplified DNA sample, for example, within a range of 3.0 × 10⁸ to 1.6 × 10¹¹ molecules/µL, or within 1.0 × 10⁹ to 1.0 × 10¹² molecules/µL, or even within 1.0 × 10¹¹ to 1.6 × 10¹¹ molecules/µL.

If qPCR is used for DNA concentration measurements, the PCR cycle number may be determined using the quantification cycle (Cq) or threshold cycle (Ct) value. An appropriate correction factor may be applied to these values based on preliminary experiments with the qPCR device and the PCR amplification device. Correction factor examples include -2, -1, 0, 1, 2, 3, or 4. Processor 13 can execute calculations based on stored qPCR data, apply the correction factor, and store the computed PCR cycle numbers in memory.

Additionally, when the DNA concentration of a DNA sample is sufficiently high, the DNA concentration can be determined by measuring absorbance using a spectrophotometer. The PCR cycle number determination device, in response to an instruction from the processor, operates a liquid transport device such as a micropipette to extract a portion of each DNA sample contained in the wells of the first multiwell plate 5. The PCR cycle number determination device then discharges a portion of the extracted DNA sample into the measurement unit of the spectrophotometer, in response to an instruction from the processor. The PCR cycle number determination device, in response to an instruction from the processor, operates the spectrophotometer and measures the absorbance of each DNA sample contained in the measurement unit. The measured absorbance is stored in the memory unit of the processor. The PCR cycle number determination device, in response to a program instruction, reads out information necessary for determining the DNA concentration from the memory unit and uses absorbance to perform a computation in the processor's calculation unit to determine the DNA concentration. The determined DNA concentration is stored in the memory unit. In this manner, the PCR cycle number determination device can determine the DNA concentration of each DNA sample contained in the wells of the first multiwell plate 5. After determining the DNA concentration, the PCR cycle number can be calculated and output in the same manner as previously described.

### PCR Cycle Number Input Step

The PCR cycle number determination step determines the PCR cycle number for each DNA sample contained in the wells of the first multiwell plate 5. The processor may store the determined PCR cycle number for each DNA sample (and thus, the PCR cycle number for each well) in the memory unit or output it to the processor of the PCR device. In this manner, the PCR cycle number for each DNA sample is input into system 1.

Figure 4 is a conceptual diagram illustrating the PCR cycle number for each DNA sample contained in the wells of the first multiwell plate. The PCR cycle number is assigned to each well. In the example shown in Figure 4, cycle numbers of 5, 6, and 7 are depicted. Once the DNA concentration of each sample is determined, the PCR cycle number determination step sets the PCR cycle number for each DNA sample (and thus, for each well). The determined PCR cycle number, along with the positional information of each sample, is input into system 1 in the PCR cycle number input step. The conceptual diagram of each DNA sample and its corresponding PCR cycle number is shown in Figure 4(a).

### Minimum PCR Step (S102)

The minimum PCR step involves performing PCR for the smallest cycle number obtained in the cycle number acquisition step (nₘᵢₙ). The individual operations in each PCR cycle follow the concept illustrated in Figure. 4.

As an example, in the process of screening peptides using the mRNA display method, when employing the DNA amplification method described in this specification, for each well of the first multiwell plate, after selecting nucleic acid-tagged peptides with the desired function from a random peptide library, the selected nucleic acids may be purified using a method such as magnetic bead-based purification, then eluted into an appropriate solvent, followed by PCR processing. Furthermore, in this case, after the purification step, the cycle number acquisition step (S101) described above may also be performed.

Prior to the minimum PCR step, it is preferable that the oil supply unit 19 supplies oil to each well of the first multiwell plate 5 (oil supply step). The oil supply unit 19 moves the transfer mechanism to the oil source based on instructions from the processor 13, and uses the aspiration and discharge mechanism to draw oil into the tube. The oil supply unit 19 then moves the tube above each well or inserts the tube into each well based on instructions from the processor 13, and discharges oil into each well using the aspiration and discharge mechanism. In this manner, the oil supply unit 19 can supply oil to each well of the first multiwell plate 5.

Prior to the minimum PCR step, it is preferable that the reaction solution supply unit 21 supplies reaction solution to each well of the first multiwell plate 5 (reaction solution supply step). In supplying this reaction solution, it is preferable that the DNA sample undergoes pre-incubation via the thermal cycler 11 before the reaction solution is supplied. The reaction solution supply unit 21 moves the transfer mechanism to the reaction solution source based on instructions from the processor 13, and uses the aspiration and discharge mechanism to draw the reaction solution into the tube. The reaction solution supply unit 21 then moves the tube above each well or inserts the tube into each well based on instructions from the processor 13, and discharges the reaction solution into each well using the aspiration and discharge mechanism. In this manner, the reaction solution supply unit 21 can supply the reaction solution to each well of the first multiwell plate 5. If the DNA sample already contains the reaction solution, the reaction solution supply step may be omitted.

The oil supply step and the reaction solution supply step are optional steps. When the oil supply step and the reaction solution supply step are performed, either may be carried out first. However, when both the oil supply step and the reaction solution supply step are performed, it is preferable to carry out the oil supply step first.

After either the oil supply step or the reaction liquid supply step, the first multiwell plate 5 may be transferred to a designated position within thermal cycler 11.

### Inner Lid Covering Step

In the minimal PCR step, it is preferable to place the inner lid 17 in a covered state (inner lid covering step). The inner lid transfer mechanism receives commands from the processor, grips the inner lid 17 using a gripper, changes the position of the gripped inner lid 17, and releases the gripper to place the inner lid 17 on top of the first multiwell plate 5. Since the inner lid 17 can be in a state covering the first multiwell plate 5, it is possible to prevent the evaporation of liquid inside each well during the PCR step.

### Movable Lid Covering Step

Prior to the minimum PCR step, it is preferable that the inner lid 17 is placed in a covered state before setting the movable lid 15 of the thermal cycler 11 to a heatable state (movable lid covering step). The drive unit changes the position of the movable lid 15 to the upper portion of the first multiwell plate 5 (therefore, if the inner lid 17 is present, to the top of the inner lid 17) based on instructions from the processor 13. In this manner, the thermal cycler 11 can prepare for the PCR step. The heating element controls the temperature based on instructions from the processor 13, thereby enabling the temperature of the sample in each well of the first multiwell plate 5 to be adjusted. Furthermore, the temperature processing time of the heating element can also be controlled by retrieving the stored PCR information from the processor's memory unit and adjusting the heating element accordingly.

### Minimal PCR Step

The minimum PCR step is a step in which PCR is performed for the minimum cycle number (nₘᵢₙ), which is the smallest cycle number obtained in the cycle number acquisition step. In other words, in this step, PCR cycles are performed nₘᵢₙ times. The polymerase chain reaction (PCR) cycles may be the same as those normally performed. Each PCR cycle typically includes the following three steps (PCR step). By repeating this cycle, the target nucleic acid in the sample can be amplified.

(1) Step 1: DNA Denaturation by Heat Treatment (Dissociation Reaction from Double-Stranded DNA to Single-Stranded DNA)
   Typically, the sample is heated to approximately 95°C.
(2) Step 2: Primer Annealing to the Template Single-Stranded DNA
   Typically, the sample temperature is set to about 55-65°C.
(3) Step 3: Primer Extension Using DNA Polymerase
   Typically, the sample is set to about 72°C.
   The temperature and processing time for each of the above steps can be adjusted appropriately using known methods. Moreover, the PCR step is not limited to the three-step PCR method mentioned above; known PCR techniques, such as two-step PCR, may also be used.

By performing the PCR cycle nₘᵢₙ times, the minimal PCR step is completed. For example, if nₘᵢₙ is 5, then the PCR step is performed in five cycles.

In the cycle number acquisition step, a reagent for terminating PCR may be added to a well containing a DNA sample that has been determined to have the minimum cycle number. This applies similarly to the following cases. The reagent for terminating PCR may be a known PCR inhibitor, such as EDTA. The step of adding the reagent for terminating PCR can be carried out in the same manner as the step of adding the reaction solution.

### First Transfer Step (S103)

The first transfer step is a step for transferring a DNA sample, which has been determined to have the minimum cycle number in the cycle number acquisition step and has undergone PCR for the minimum number of cycles, to the second multiwell 7. At this time, it is preferable that the transferred DNA sample is moved to a well in the second multiwell 7 that corresponds to the well in the first multiwell 5. As previously described, it is desirable that the second multiwell 7 is kept stationary under cooling conditions.

When a movable lid 15 or an inner lid 17 is used in the PCR step, it is preferable that, in the first transfer step, the movable lid 15 is set to a non-heating state, and the inner lid 17 is set to an open state.

### Movable Lid Initialization Step

When the movable lid 15 is used in the PCR step, the drive unit changes the position of the movable lid 15 from above the first multiwell 5 to its initial position based on a command from the processor. An example of the initial position is inside the thermal cycler 11. At this time, the upper part of the thermal cycler 11 is in an open state. In this way, the thermal cycler can set the movable lid 15 to a non-heating state (movable lid initialization step). As previously described, it is preferable that the movable lid 15 has a heating element.

### Inner Lid Opening Step

When performing PCR using the inner lid 17, the inner lid transfer mechanism receives a command from the processor 13, grips the inner lid 17 located above the first microwell 5 using a gripper, changes the position of the gripped inner lid 17 to the inner lid initial position, and releases the gripper, thereby placing the inner lid 17 in the inner lid initial position. In this way, the inner lid transfer mechanism can set the inner lid 17 to an open state.

In the first transfer step, it is preferable to transfer the sample, which is the target of the first transfer step, from the well of the first multiwell 5 to the corresponding well of the second multiwell 7.

As one mode of the transfer mechanism of the sample transport unit 9 in the first transfer step, an automatic pipetting device can be used, but the typical functionality of an automatic pipetting device involves adding different liquid volumes to each well or aspirating different liquid volumes for collection into another multiwell plate and it is not designed to selectively extract irregularly positioned samples from a single plate, interpose another operation, and then continue the collection step in repeated steps.

Here, the processor 13 stores the positional information of the wells in the first multiwell 5 in which PCR is performed for the minimum number of cycles (nₘᵢₙ), as well as the corresponding well positions in the second multiwell 7. The transfer mechanism of the sample transport unit 9 receives commands from the processor and moves the tube position to the wells in the first multiwell 5 where PCR has been conducted for the minimum number of cycles (nₘᵢₙ).

Then, the aspiration and discharge mechanism of the sample transport unit 9 receives commands from the processor 13 and aspirates the sample in the wells where PCR has been completed for the minimum number of cycles (nₘᵢₙ). The transfer mechanism of the sample transport unit 9 receives commands from the processor 13 and moves the tube position to the corresponding wells in the second multiwell 7 where PCR has been performed for the minimum number of cycles (nₘᵢₙ). The transfer mechanism of the sample transport unit 9 may insert the tube into the well if necessary.

The aspiration and discharge mechanism of the sample transport unit 9 receives commands from the processor 13 and discharges the sample into the wells of the second multiwell 7. In this manner, the system 1 can transfer the sample, which is the target of the first transfer step, from the well of the first multiwell 5 to the corresponding well of the second multiwell 7. If the well contains oil, it is preferable that the aspiration and discharge mechanism of the sample transport unit 9 receives commands from the processor 13 and is controlled to aspirate the liquid in the well at a suction force sufficient to avoid aspirating the oil while extracting the DNA-containing liquid. In this way, components other than oil can be aspirated. In this case, it is preferable that the transfer mechanism of the sample transport unit 9 receives commands from the processor 13 and moves the tube position to a predetermined position (predetermined height position) within the wells of the first multiwell 5 where PCR is performed for the minimum number of cycles (nₘᵢₙ).

In the example of FIG. 4, after performing five cycles of PCR, the DNA sample contained in the wells with a PCR cycle number of five is transferred to the corresponding wells of the second multiwell plate. FIG. 4(b) illustrates the state where the DNA sample from a well with a cycle number of five has been transferred to the corresponding well of the second multiwell plate.

### Iterative Additional PCR and Transfer Step (S104)

The repeated additional PCR and transfer step is a step in which, after the first transfer step, PCR is further performed up to the maximum cycle number, which is the greatest cycle number acquired in the cycle number acquisition step, and samples that have reached the cycle number acquired in the cycle number acquisition step are repeatedly transferred to the second multiwell 7. The temperature and processing time of each step in the PCR cycle can be appropriately adjusted based on known methods, but it is preferable to use the same conditions as the minimum PCR step.

For example, in performing the (nₘᵢₙ + 1)-th PCR cycle, system 1 may proceed directly to the PCR step after the first transfer step. Additionally, system 1 may perform the inner lid covering step and the movable lid covering step, followed by executing the PCR step once.

If there is no DNA sample in the wells of the first multiwell 5 with a PCR cycle number of (nₘᵢₙ+1), the processor 13 instructs system 1 to perform the (nₘᵢₙ+2)th PCR cycle. In this case, the processor 13 retrieves PCR information from the memory unit and performs a calculation to determine whether there is a DNA sample with a PCR cycle number of (nₘᵢₙ+1). If no such sample exists, the processor 13 outputs a command instructing system 1 to perform the (nₘᵢₙ+2)th PCR cycle.

If there is a DNA sample in the wells of the first multiwell 5 with a PCR cycle number of (n_min+1), the second transfer step is performed. At this time, system 1 may perform the movable lid initialization step and the inner lid opening step before executing the second transfer step.

The processor 13 stores the positional information of the wells in the first multiwell 5 where PCR is performed for (n_min+1) cycles, as well as the positional information of the corresponding wells in the second multiwell 7. Therefore, the processor 13 can use system 1 to transfer the DNA samples contained in the wells of the first multiwell 5, where PCR has been performed for (n_min+1) cycles, to the corresponding wells in the second multiwell 7. This step is the same as the first transfer step.

The maximum cycle number, which is the greatest cycle number acquired in the cycle number acquisition step, is defined as (nₘₐₓ). The repeated additional PCR and transfer step continues from the (nₘᵢₙ+1)th PCR step to the (nₘₐₓ)th PCR step, repeating the previously mentioned step. In this manner, each well of the second multiwell 7 will contain DNA samples with a concentration within a specified range.

In the example of Figure 4, a 5-cycle PCR is performed, and after transferring the DNA sample contained in the wells with a PCR cycle number of 5 to the corresponding wells in the second multiwell, the inner lid covering step and the movable lid covering step are carried out, followed by the PCR cycle. As a result, the PCR cycle number increases by one, reaching 6. In the example of Figure 4, there are wells with a PCR cycle number of 6. Therefore, after performing the 6-cycle PCR, the movable lid initialization step and the inner lid opening step are carried out. Then, the second transfer step is performed for the wells with a PCR cycle number of 6. Next, in the example of Figure 4, the inner lid covering step and the movable lid covering step are performed, followed by the PCR cycle. As a result, the PCR cycle number increases by one, reaching 7. In the example of Figure 4, there are wells with a PCR cycle number of 7. Therefore, after performing the 7-cycle PCR, the movable lid 15 initialization step and the inner lid 17 opening step are carried out. Then, the third transfer step is performed for the wells with a PCR cycle number of 7. As a result, DNA samples with a concentration within a specified range are contained in the wells of the second multiwell 7 corresponding to the wells of the first multiwell 5.

Since the method of the present invention includes the repeated additional PCR and transfer steps, the thermal cycler repeatedly switches between open and closed states.

The sample transport unit, oil supply unit, and reaction liquid supply unit may all be separate devices, or any two of them, or all of them may be the same device. Since all of these units include a tube such as a pipette (micropipette), a transfer mechanism for moving the tube, and a aspiration and discharge mechanism for adjusting the air inside the tube to enable aspiration and discharge, the same device can be used to perform the sample transport step, oil supply step, and reaction liquid supply step by simply replacing the pipette tip. Each step switch can be appropriately executed based on instructions from the processor.

System 1 can amplify the DNA concentration of multiple different DNA samples within a specified range without dilution, making it suitable for the amplification of DNA samples recovered in in vitro displays such as mRNA display and RAPID display. This is because it allows DNA amplification while maintaining diversity, enabling a larger number of candidate peptides to be subjected to the selection step.

### Example 1

The system comprises a computer, exchangeable pipette tips, an automatic dispenser, a micropipette (sample transport unit), a collection multiwell plate, a magnet plate, a thermal cycler (temperature control unit), a PCR multiwell plate, an inner lid storage area, a robotic arm, an inner lid, a thermal cycler's movable lid, an oil source, and a reaction liquid source.

An example configuration of the DNA amplification system of the present invention is as follows.
The first and second multiwell plates used are 96-well multiwell plates (manufactured by Thermo Fisher Scientific).

The sample transport unit utilizes the TECAN Fluent^{®} Automation WORKSTATION, which includes an automatic pipetting device and a robotic arm. The automatic pipetting device built into this WORKSTATION has eight independent pipetting channels, allowing for pipetting operations and pipette transfer operations.

Furthermore, this WORKSTATION is also used as the oil supply unit and reaction liquid supply unit. By appropriately replacing the pipette tips, each function can be performed. The WORKSTATION also has 96 fixed pipetting channels, which are suitable for supplying reaction liquids by dispensing into all wells simultaneously. Instead of the TECAN WORKSTATION, it is also possible to use TECAN's Freedom EVO series, Hamilton's Vantage series, or Beckman Coulter's Biomek series WORKSTATION.

For the thermal cycler, the Inheco On-Deck Thermal Cycler (ODTC) is used. This thermal cycler has a movable heated lid, allowing automatic control of lid heating and opening/closing operations. Alternatively, Jena's T Robot or Thermo Fisher Scientific's ATC can also be used.

The inner lid used is the Auto-Sealing PCR Plate Lid from Azenta Life Sciences. Alternatively, the arch-shaped Auto-Sealing Lid #MSL2022 from Bio-Rad can be used. For the oil source and reaction liquid source, the 100 mL Trough from TECAN is used.
All of the above components are arranged as shown in Figure 1A.

The WORKSTATION and thermal cycler are connected to a computer, and the FluentControl^{™} software stored in the computer enables coordinated control of the automatic pipetting device, robotic arm, and thermal cycler. The computer stores a program that implements the method of the present invention, allowing each component to operate accordingly.

Additionally, the LightCycler^{®} 96 from Roche Diagnostics is used as the thermal cycler for quantitative PCR.

Using the DNA amplification system configured as described above, DNA amplification can be performed as follows.

A random DNA library is created, containing a DNA sequence with 30 to 45 randomized nucleotides between a nucleic acid coding for a T7 promoter sequence at the 5' end of the DNA sense strand and a linker annealing site coding for puromycin and a selectable peptide at the 3' end. Using this, a random peptide library is generated according to the TRAP display method (WO2014/119600), where each peptide has a DNA/RNA heteroduplex nucleic acid tag. Each generated random peptide library is introduced into a multiwell plate containing magnetic beads immobilized with the desired target protein in each well. Peptides with nucleic acid tags bound to the magnetic beads are then recovered using a magnet.

The nucleic acid encoding the peptide sequence bound to the target protein is treated with 50 µL of PCR reaction solution and processed at 94°C, then recovered in a new multiwell plate (first multiwell) and used as DNA samples for the PCR step described in this patent. The PCR reaction solution used at this time contains primers at a final concentration of 0.25 µM, dNTPs (deoxynucleoside triphosphates) as substrates for ATGC at 0.25 mM, 1× PCR buffer, 2 mM MgCl₂, and salts such as KCl. The primers used are as follows: the forward primer is a 52-base nucleic acid that anneals to the T7 promoter sequence at the 5' end of the DNA, and the reverse primer is a 44-base nucleic acid that anneals to the nucleic acid coding for the linker connecting puromycin and the peptide at the 3' end of the DNA.

From each well in the multiwell plate, 1 µL of each DNA sample is extracted, and its concentration is analyzed using quantitative PCR (qPCR). The PCR cycle number for each DNA sample is determined by adding 2 to the acquired Ct value. This added value is determined based on preliminary studies conducted using the PCR device for quantitative PCR and the PCR device for amplification. In this case, the PCR cycle numbers acquired from the current samples are assumed to be 5, 6, and 7. The PCR cycle numbers are entered into the computer system, and the program of the present invention is executed.

Using the above automatic pipetting device, mineral oil stored in the oil source is supplied to each well in an appropriate amount to suppress evaporation of the reaction liquid. Next, the multiwell plate (first multiwell) containing each DNA sample is transferred onto the thermal cycler using a robotic arm, and the inner lid, placed in the inner lid storage, is also transferred by the robotic arm and placed over the multiwell plate (first multiwell) set on the thermal cycler to cover it. Then, the movable lid is set to a heating-enabled state that covers the first multiwell, followed by pre-incubation at 94°C. After pre-incubation, the Taq polymerase (reaction liquid in the present invention) stored in the reaction liquid source is supplied to each well at a final concentration of 25 Units/mL using the automatic pipetting device.

PCR is performed for five cycles, which is the lowest cycle number among the DNA samples. The PCR conditions can be selected as needed, for example, 94°C for 40 seconds, 61°C for 40 seconds, and 72°C for 40 seconds.

After completing the five-cycle PCR, the position of the movable lid is moved from above the first multiwell to its initial position, setting it to a non-heating state. Next, the inner lid is transferred using the robotic arm and placed in the inner lid storage (inner lid initial position).

Next, the DNA sample that has undergone five PCR cycles (Cycle 5 in Figure 4(b)) is collected using an automatic pipetting device and transferred to the corresponding well of the first multiwell in a multiwell plate (second multiwell) mounted in a collection sample loading unit equipped with a cooling mechanism utilizing a circulator.

After transferring the DNA samples from the five-cycle PCR, the inner lid stored in the inner lid storage is transferred using the robotic arm and placed over the multiwell plate (first multiwell plate) on the thermal cycler to cover it. Next, the movable lid is set to a heating-enabled state that covers the first multiwell plate. Then, the sixth PCR cycle is performed. The PCR conditions can be appropriately selected, but for example, the same conditions as the five-cycle PCR may be used.

After completing the sixth PCR cycle, the position of the movable lid is moved from above the first multiwell plate to its initial position, setting it to a non-heating state. Next, the inner lid is transferred using the robotic arm, then placed in the inner lid storage. Then, the DNA sample that has undergone six PCR cycles is collected using an automatic pipetting device and transferred to the well of the multiwell plate (second multiwell plate), corresponding to the well of the first multiwell plate.

After transferring the DNA sample from the six-cycle PCR, the inner lid stored in the inner lid storage is transferred using the robotic arm and placed over the multiwell plate (first multiwell) on the thermal cycler to cover it. Then, the movable lid is set to a heating-enabled state that covers the first multiwell, followed by performing the seventh PCR cycle. The PCR conditions can be appropriately selected, but for example, the same conditions as the five-cycle PCR may be used.

After completing the seventh PCR cycle, the position of the movable lid is moved from above the first multiwell plate to its initial position, setting it to a non-heating state. Next, the inner lid is transferred using the robotic arm, then placed in the inner lid storage. Then, the DNA sample that has undergone seven PCR cycles is collected using an automatic pipetting device and transferred to the well of the multiwell plate (second multiwell plate), corresponding to the well of the first multiwell plate.

Through these steps, all DNA samples are transferred from the first multiwell plate to the corresponding wells of the second multiwell plate, completing the amplification step.
This amplification step ensures that the DNA concentration of each amplified sample remains within a specified range without losing the diversity of the target DNA sample.

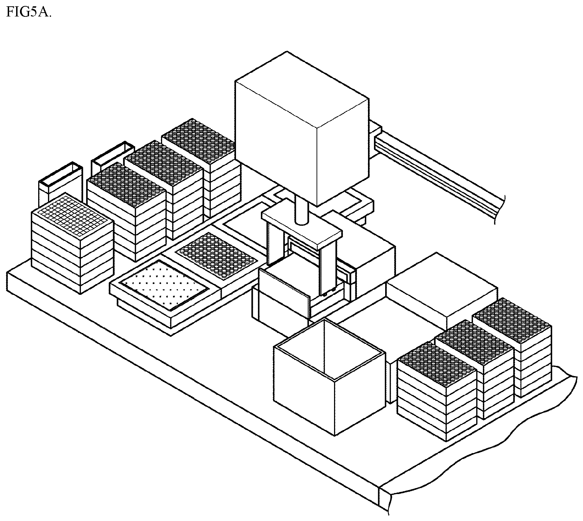

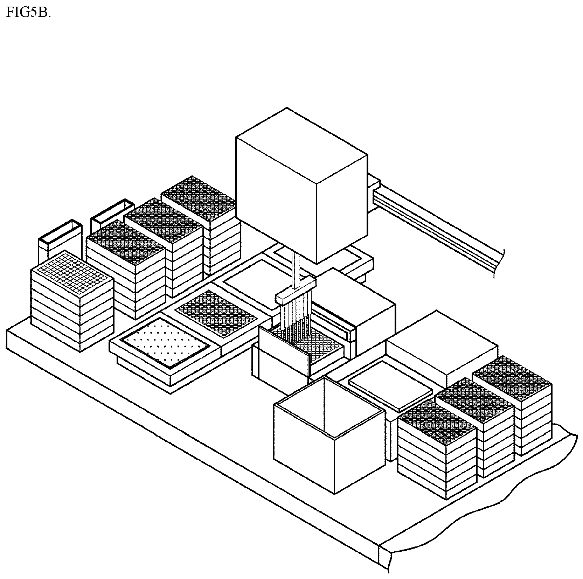

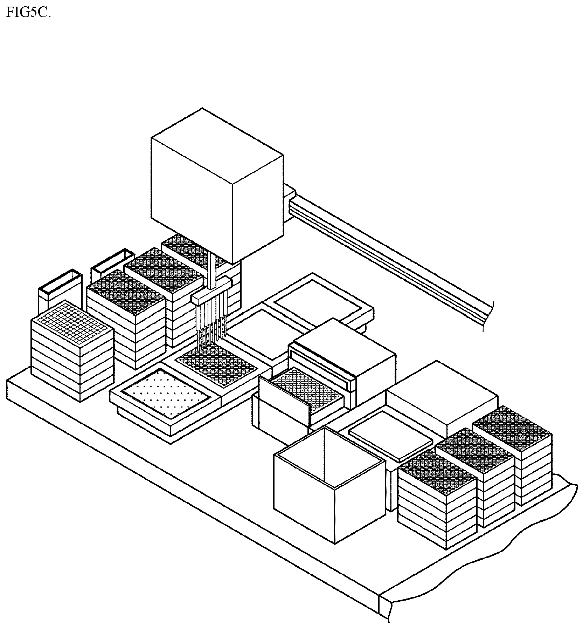

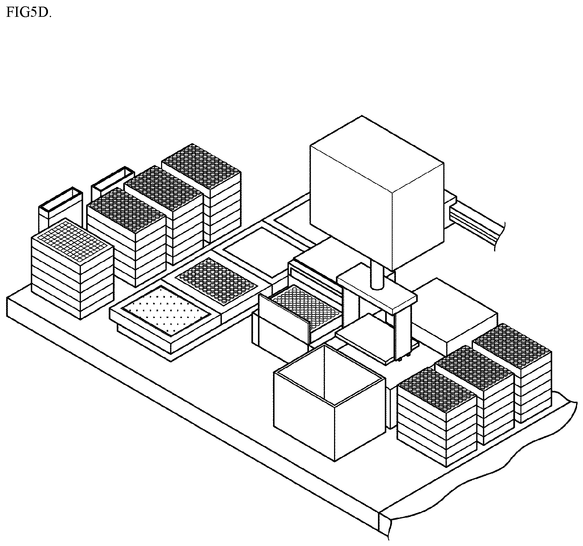

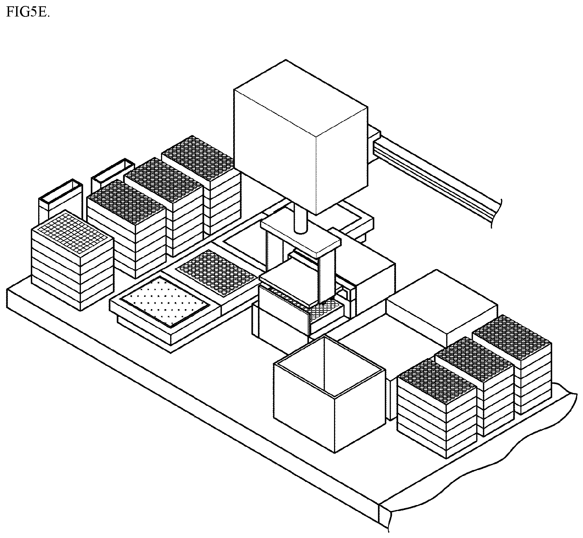

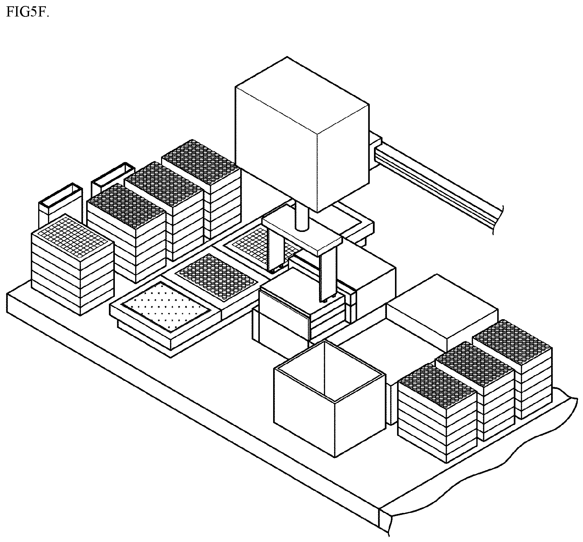

FIG. 5 is a set of schematic diagrams illustrating the operation of the PCR device in this embodiment.

FIG. 5A shows a conceptual diagram where the inner lid is lifted. As depicted, the movable lid of the thermal cycler slides open, after which the robotic arm lifts the inner lid and transports it to the inner lid storage area.

FIG. 5B illustrates the collection of DNA samples that have completed their designated PCR cycles. The automatic dispenser collects the samples from the wells of the multiwell plate placed in the thermal cycler. In the example shown, multiple samples are collected simultaneously.

FIG. 5C depicts the step of transferring the collected DNA samples to the corresponding wells of the second multiwell plate. As shown, the samples are discharged into the appropriate wells.

FIG. 5D shows the step of transferring the inner lid back to the thermal cycler after DNA sample collection. The robotic arm moves the inner lid from the storage area to the multiwell plate in the thermal cycler.

FIG. 5E is a conceptual diagram illustrating the transfer of the inner lid onto the thermal cycler. As shown in Figure 5E, the inner lid is placed on the multiwell plate set on the thermal cycler.

FIG. 5F shows the thermal cycler's movable lid transitioning into a heatable state. The movable lid slides shut, and once fully closed, the next PCR cycle begins.

### Example 2

In this embodiment, multiple DNA samples with different concentrations were prepared, and these DNA samples were used as model samples to examine whether the amplification method of the present invention could adjust the DNA concentration of each sample within a defined range.

### DNA Amplification System Configuration

The configuration of the DNA amplification system for supplying DNA samples was as follows:
The first and second multiwells used 96-well multiwell plates from Thermo Fisher Scientific.

The sample transport unit utilized the TECAN Fluent^{®} Automation WORKSTATION, which includes an automatic pipetting device and a robotic arm.

Furthermore, this WORKSTATION was also used as the oil supply unit and reaction liquid supply unit. An 8-channel automatic pipetting device was used for oil supply, and a 96-channel automatic pipetting device was used for reaction liquid supply.
The On-Deck Thermal Cycler (ODTC) from Inheco was used as the thermal cycle.
The Auto-Sealing PCR Plate Lid from Azenta Life Sciences was used as the inner lid.
The 100 mL Trough from TECAN was used as the oil source and reaction liquid source.

All components were arranged as shown in FIG. 1A.
The WORKSTATION and thermal cycler were connected to a computer, and the FluentControl^{™} software stored in the computer was configured to enable coordinated control of the automatic pipetting device, robotic arm, and thermal cycler. The computer was programmed with software implementing the method of the present invention, allowing each component to operate accordingly.

Additionally, the LightCycler^{®} 96 from Roche Diagnostics was used as the thermal cycler for quantitative PCR.

### DNA Amplification Step

Using the configured DNA amplification system, DNA amplification was conducted as follows:
The DNA described in Sequence ID No. 1 was used as a template for PCR amplification. Template DNA (Sequence ID No. 1) at a concentration of approximately 3×10¹⁰ molecular/µL was first diluted six fold with PCR reaction solution and then further diluted in eight steps at a ratio of 2×, preparing eight samples with different concentrations. These samples were randomly placed in a 96-well multiwell plate (first multiwell plate) and used as DNA samples for the PCR step. The PCR reaction solution used for the following quantitative PCR and amplification PCR contained primers at a final concentration of 0.25 µM, dNTPs (deoxynucleoside triphosphates) as substrates for ATGC at 0.25 mM, 1× PCR buffer, and 2 mM MgCl₂. The primers used were as follows: the forward primer was a nucleic acid of a specified length annealing to the 5' end of the DNA (Sequence ID No. 2), and the reverse primer was a nucleic acid of a specified length annealing to the 3' end of the DNA (Sequence ID No. 3).

Template DNA (5'–

3')GGCGTAATACGACTCACTATAGGTGGGGGTTCCCGAGCGGCCAAAAGGGAGCAGA

CTGTAAAATCTGCCGTCACAGACTTCGAAGGTTCGAATCCCTTCCCCCACCACCA

(Sequence ID No. 1)

Forward primer (5'-3') CCGCGTAATACGACTCACTATAG (Sequence ID No. 2) Reverse primer (5'-3') TG(M)GTGGGTGGGGGAAGGATTCG (Sequence ID No. 3) (M) represents a 2'-methoxy modification of the preceding nucleic acid.

From each well of the multiwell plate (first multiwell), 1 µL of each DNA sample was extracted, and its concentration was analyzed using quantitative PCR (qPCR), with the acquired Ct value rounded and then increased by 2 to determine the PCR cycle number for each DNA sample. As a result, the minimum PCR cycle number was 6, and the maximum was 20. The PCR cycle numbers for each DNA sample are shown in Table 1. This added value was determined based on preliminary studies conducted using the PCR device for quantitative PCR and the PCR device for amplification.

**Table 1**

| | 1 | 2 | 3 | 4 | 5 | 6 | 7 | 8 | 9 | 10 | 11 | 12 |
|---|---|---|---|---|---|---|---|---|---|---|---|---|
| A | 9 | 14 | 12 | 12 | 19 | 10 | 10 | 16 | 15 | 13 | 11 | 7 |
| B | 13 | 19 | 16 | 16 | 8 | 10 | 14 | 18 | 8 | 14 | 11 | 9 |
| C | 9 | 7 | 6 | 18 | 16 | 17 | 16 | 14 | 6 | 15 | 12 | 18 |
| D | 14 | 8 | 17 | 9 | 18 | 6 | 6 | 13 | 11 | 8 | 17 | 16 |
| E | 17 | 17 | 11 | 11 | 18 | 6 | 11 | 18 | 13 | 9 | 11 | 16 |
| F | 6 | 11 | 8 | 8 | 13 | 6 | 15 | 14 | 6 | 8 | 17 | 14 |
| G | 14 | 11 | 8 | 13 | 6 | 11 | 19 | 14 | 11 | 10 | 15 | 8 |
| H | 14 | 19 | 11 | 18 | 7 | 16 | 16 | 19 | 19 | 20 | 20 | 17 |

The acquired PCR cycle numbers and the corresponding well position information containing the samples were entered into the computer of the system, and the program of the present invention was executed.

Using the automatic pipetting device, mineral oil stored in the oil source was supplied to each well in an appropriate amount to suppress evaporation of the reaction liquid. Next, the multiwell plate (first multiwell plate) containing each DNA sample was transferred onto the thermal cycler using a robotic arm, and the inner lid placed in the inner lid storage was transferred by the robotic arm and placed over the multiwell plate (first multiwell plate) set on the thermal cycler to cover it. Then, the movable lid was set into a heatable state, and pre-incubation at 94°C was performed.

After pre-incubation, the Taq polymerase (reaction liquid in the present invention) stored in the reaction liquid source was supplied to each well at a final concentration of 25 Units/mL using the automatic pipetting device. PCR was performed for six cycles, which is the minimum cycle number among the DNA samples. The PCR conditions were set to 94°C for 40 seconds, 61°C for 40 seconds, and 72°C for 40 seconds.

After completing the six-cycle PCR, the position of the movable lid was moved from above the first multiwell plate to its initial position, setting it to a non-heating state. Next, the inner lid was transferred using the robotic arm and placed in the inner lid storage (inner lid initial position). Then, the DNA samples that underwent six PCR cycles were recovered using the automatic pipetting device and transferred to the corresponding wells of the second multiwell plate in a collection sample loading unit equipped with a cooling mechanism using a circulator.

After transferring the DNA samples from the six-cycle PCR, the inner lid stored in the inner lid storage was transferred using the robotic arm and placed over the multiwell plate (first multiwell plate) on the thermal cycler to cover it. Then, the movable lid was set to a heating-enabled state that covers the first multiwell plate, followed by performing the seventh PCR cycle. The PCR conditions were set to be the same as those for the six-cycle PCR. From the seventh PCR cycle onward, the same procedure as in the sixth cycle was followed, transferring the DNA samples from each PCR cycle from the multiwell plate (first multiwell plate) to the multiwell plate (second multiwell plate), repeating the step until the maximum cycle number of 20 cycles was reached.

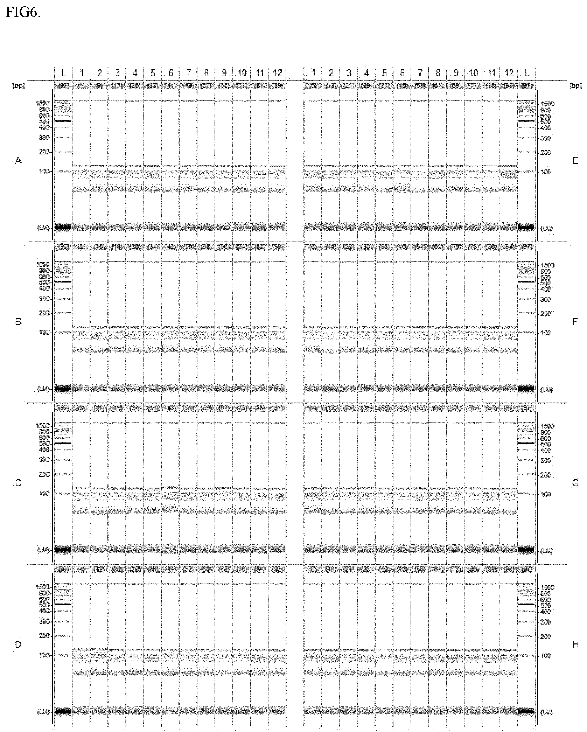

Through these steps, all DNA samples were transferred from the first multiwell plate to the corresponding wells of the second multiwell plate, completing the amplification step. The DNA amplification of the DNA samples following the completed amplification step was confirmed using a microfluidic electrophoresis device (MultiNA, Shimadzu) (Figure 6). Figure 6 is a photograph substituting for a drawing that illustrates the microfluidic electrophoresis image in Example 2. The labels A-H and 1-12 in Figure 6 correspond to A-H and 1-12 in Table 1, respectively. The DNA targeted for PCR amplification was approximately 106 bp, and its band was observed at around 106 bp in the image. Furthermore, the concentration of all samples fell within the range of 8.86 × 10⁹ to 5.27 × 10¹⁰ molecules/µL. Based on the above results, it was confirmed that the amplification step of the present invention enabled multiple DNA samples with varying concentrations to maintain their DNA concentration within a defined range after PCR amplification.

### INDUSTRIAL APPLICABILITY

This invention can be utilized in industries related to pharmaceuticals and biotechnology.

### [REFERENCE SIGNS LIST]

- 1 -: DNA amplification system
- 3 -: PCR device
- 5 -: First multiwell plate
- 7 -: Second multiwell plate
- 9 -: Sample transport unit
- 11 -: Thermal cycler
- 13 -: Processor
- 15 -: Movable lid
- 17 -: Inner lid
- 19 -: Oil supply unit
- 21 -: Reaction liquid supply unit
- 23 -: Magnet for magnetic bead collection
- 31 -: Input unit
- 33 -: Output unit
- 35 -: Control unit
- 37 -: Computation unit
- 39 -: Storage unit
- 41 -: Bus

## Claims

1. A method of amplifying DNA using PCR, comprising:
a cycle number acquisition step of acquiring a PCR cycle number for each DNA sample contained in each well of a first multiwell plate;
a minimum PCR step of performing PCR for a minimum number of cycles, which is the smallest number of cycles acquired in the cycle number acquisition step;
a first transfer step of transferring a sample, which has been identified as having the minimum cycle number in the cycle number acquisition step and underwent PCR for the minimum number of cycles, to a second multiwell plate; and
a step of repeatedly performing an additional PCR and transfer step, wherein, after the first transfer step, PCR is further performed up to a maximum number of cycles, which is the greatest number of cycles acquired in the cycle number acquisition step, and a sample that has reached the number of cycles acquired in the cycle number acquisition step is transferred to the second multiwell plate.

2. The method according to claim 1, wherein the cycle number acquisition step includes acquiring a PCR cycle number such that a concentration of amplified DNA falls within a predetermined range based on the DNA concentration of the each DNA sample contained in the well.

3. The method according to claim 1, wherein the each DNA sample in the each well contains multiple types of DNA, and the multiple types of DNA share identical base sequences at both 5' and 3' ends.

4. The method according to claim 1, using a movable lid and an inner lid, wherein
the movable lid can be set to a heatable state to cover the first multiwell plate, and to a non-heating state to uncover the first multiwell plate by moving away from the heatable state;
the inner lid can be set to a covered state to cover the first multiwell plate, and to an open state to uncover the first multiwell plate by moving away from the covered state; when the movable lid is in the heatable state and the inner lid is in the covered state, the inner lid is positioned between the movable lid and the first multiwell plate;
in the minimum PCR step, the inner lid is set to the covered state and the movable lid is set to the heatable state;
in the first transfer step, the movable lid is set to the non-heating state and the inner lid is set to the open state;
in the additional PCR and transfer step, when PCR is being performed, the inner lid is set to the covered state and the movable lid is set to the heatable state; and
in the additional PCR and transfer step, when transferring the sample from the first multiwell plate to the second multiwell plate, the movable lid is set to the non-heating state and the inner lid is set to the open state.

5. The method according to claim 1, wherein
each well of the second multiwell plate corresponds to each well of the first multiwell plate;
in the first transfer step, the sample to be transferred is transferred from the well of the first multiwell plate to the well of the second multiwell plate, corresponding to the well of the first multiwell plate; and
in the additional PCR and transfer step, when transferring the sample from the first multiwell plate to the second multiwell plate, the sample to be transferred is transferred from the well of the first multiwell plate to the well of the second multiwell plate, corresponding to the well of the first multiwell plate.

6. The method according to claim 1, further comprising:
a step, prior to the minimum PCR step, in which an oil supply unit supplies oil to each well of the first multiwell plate; and
a step, prior to the minimum PCR step, in which a reaction liquid supply unit supplies reaction liquid to each well of the first multiwell plate.

7. A program for amplifying DNA using a PCR apparatus including a processor, the program causing the processor to:
execute a cycle number input step in which a PCR cycle number for each DNA sample in each well of a first multiwell plate of the PCR apparatus is input;
a minimum PCR step in which the PCR apparatus performs PCR for a minimum number of cycles, which is the smallest number of cycles input in the cycle number input step;
a first transfer step in which the PCR apparatus transfers a sample, which was identified as having the minimum number of cycles of PCR and underwent PCR to a second multiwell plate; and
a step in which the PCR apparatus repeatedly performs an additional PCR and transfer step, wherein, after the first transfer step, PCR is further performed up to a maximum number of cycles, which is the greatest number of cycles acquired in the cycle number input step, and a sample that has reached the number of cycles input in the cycle number input step is transferred to the second multiwell plate.

8. A non-transitory information storage medium storing the program according to claim 7.

9. A DNA amplification system using a PCR apparatus comprising a first multiwell plate, a second multiwell plate, a sample transfer unit, a thermal cycler, and a processor, the processor being configured to:
execute a cycle number input step in which a PCR cycle number for each DNA sample in the first multiwell plate of the PCR apparatus is input;
a minimum PCR step in which the processor drives the thermal cycler equipped with the first multiwell plate and causes the PCR apparatus to perform PCR for a minimum number of cycles, which is the smallest number of cycles input in the cycle number input step;
a first transfer step in which the processor uses the sample transfer unit to transfer a sample, which was identified as having a minimum number of cycles of PCR and underwent PCR for the minimum cycle number, to the second multiwell plate; and
a step in which the processor drives the thermal cycler equipped with the first multiwell plate and repeatedly performs an additional PCR and transfer step in which after the first transfer step, PCR is further performed up to a maximum number of cycles, which is the greatest number of cycles acquired in the cycle number input step, and a sample that has reached the number of cycles input in the cycle number input step is transferred to the second multiwell plate.

10. The system according to claim 9, further comprising a movable lid and an inner lid, wherein:
the movable lid can be set to a heatable state to cover the first multiwell and a non-heating state to uncover the plate by moving away from the heatable state;
the inner lid can be set to a covered state to cover the first multiwell and an open state to uncover the first multiwell plate by moving away from the covered state;
when the movable lid is in the heatable state and the inner lid is in the covered state, the inner lid is positioned between the movable lid and the first multiwell plate;
in the minimum PCR step, the processor sets the inner lid to the covered state and the movable lid to the heatable state;
in the first transfer step, the processor sets the movable lid to the non-heating state and the inner lid to the open state;
in the additional PCR and transfer step while PCR is being performed, the processor sets the inner lid to the covered state and the movable lid to the heatable state;
and in the additional PCR and transfer step, when transferring the sample from the first multi-well to the second multi-well, the processor sets the movable lid to the non-heating state and the inner lid to the open state.

11. The system according to claim 9, wherein:
each well of the second multiwell plate corresponds to each well of the first multiwell plate;
in the first transfer step, the processor causes the sample to be transferred from the well of the first multiwell plate to the well of the second multiwell plate, corresponding to the well of the first multiwell plate; and
in the additional PCR and transfer step, when transferring the sample from the first multiwell plate to the second multiwell plate, the processor causes the sample to be transferred from a well of the first multiwell plate to the well of the second multiwell plate, corresponding to the well of the first multiwell plate.

12. The system according to claim 9, further comprising:
an oil supply unit configured to supply oil to each well of the first multiwell plate; and
a reaction liquid supply unit configured to supply reaction liquid to each well of the first multiwell plate,
wherein, prior to the minimum PCR step, the processor controls the oil supply unit to supply oil and the reaction liquid supply unit to supply reaction liquid to the wells of the first multiwell plate.
